# EUROPEAN PATENT APPLICATION

(11) **EP 1 008 995 A1**
(43) Date of publication of application: **14.06.2000**
(21) Application number: 99250374.8
(22) Date of filing: 21.10.1999
(51) Int. Cl.: G21G 4/08

(54) **Medical radioactive palladium-103 miniature radiation sources and methods of producing same**

(30) Priority: 12.12.1998 DE 19859101; 12.12.1998 DE 19859100
(71) Applicant: Eurotope Entwicklungsgesellschaft für Isotopentechnologien mbH, 13125 Berlin (DE)
(72) Inventor: Hess, André, 13053 Berlin (DE); Mann, Gunnar, 13125 Berlin (DE); Krutjakow, Alexei, 14169 Berlin (DE); Ziegler, Jürgen, 12679 Berlin (DE); Pallas, Rolf, 08412 Leubnitz (DE)
(74) Representative: Ziebig, Marlene, Dr. Dipl.-Chem.

(57) **Abstract**

The invention relates to new radioactive palladium-103 miniature radiation sources (seeds) wherein the carrier matrix consists of a porous and mechanically stable inorganic material, the pores of which contain Pd-103 as metal or in the form of a stable and water-insoluble Pd-103 compound. The activity carrier matrix is encapsulated in a corrosion-resistent and body-compatible material or coated with such a material. The palladium-103 miniature radiation sources of the invention may contain (a) conventional X-ray marker(s). The invention is also directed to methods of producing said new palladium-103 miniature radiation sources.

## Description

The invention relates to new radioactive palladium-103 miniature radiation sources (seeds) comprising a radioactive carrier matrix which consists of a porous and mechanically stable inorganic material, the pores of which contain Pd-103 as metal or in the form of a stable and water-insoluble Pd-103 compound. The radioactive carrier matrix is encapsulated in a corrosion-resistent and body-compatible material or coated with such a material. The palladium-103 miniature radiation sources of the invention may contain (a) conventional X-ray marker(s). The invention is also directed to methods of producing said new palladium-103 miniature radiation sources.

The main field of use for these palladium miniature radiation sources is in the treatment of tumors. Interstitial therapy is possible as a preferred method, wherein the radiators are implanted in the tissue to be treated. The small size and the short half-life of the nuclide allows permanent residence in the tissue.

Local irradiation of tumors using interstitial implants has been conventional practice for quite some time. Such a treatment can be dosed precisely and limited locally. The effects on healthy tissue are minimized.

The palladium-103 isotope has an average gamma energy of 20-23 keV and a physical half-life of 17 days. As a result, it is well suited for interstitial brachytherapy. Irradiation is largely restricted to the tumor, and healthy tissue is not substantially affected. The medical personnel's radiation exposure is minimal.

The construction of the miniature radiation sources must allow rapid and easy introduction of same into the tissue to be treated. One common technique of introduction is the use of hollow needles. Using these, the desired number of miniature radiation sources are positioned in the tissue, and the needle is withdrawn.

To identify and position the radiation sources in the body, soft X-rays are preferably used in diagnostics. Therefore, the radiation sources should comply with the fundamental requirement of comprising an element of high atomic number and density, which is visible in X-ray diagnostics. In addition, such a so-called "X-ray marker" should be of a nature so as to provide information on the spatial arrangement of the miniature radiation source. To date, gold, silver, platinum, lead, iridium, or other high density metals have been used as materials. On the one hand, shape and size of the X-ray markers depend on the configuration and size of the activity carrier and, on the other hand, on imaging requirements. Thus, the X-ray markers can be beads, wires or tubes.

In the literature, preferably in the patent literature, a number of miniature radiation sources for brachytherapy have been described, which are frequently referred to as "seeds". As nuclides, they contain low-energy gamma radiators, preferably such as iodine-125 or palladium-103 [US patents Nos. 5,405,165 (Carden); 5,354,257 (Roubin); 5,342,283 (Good); 4,891,165 (Suthanthiryan); 4,702,228 (Russel et al.); 4,323,055 (Kubiatowicz), 3,351,049 (Lawrence) and WO 97/19706 (Coniglione)].

Thus, for example, Lawrence (US 3,351,049) describes cylindrical seeds containing plastic bodies or nylon bodies as activity carrier, onto which nuclides such as I-125 or Pd-103 have been applied. The iodine-125 seeds described by Kubiatowicz (US 4,323,055) have a radioactive iodine layer on the surface of a silver rod. These seeds are produced by conversion of the silver surface of the silver rod to silver chloride and subsequent exchange reaction using iodide-125. The silver rod simultaneously serves as an X-ray marker. As a result of the large diameter of the rod, virtually all of the interior of the sealed capsule is filled with silver, so that self-absorption of the soft gamma radiation occurs to a large extent. In order to obtain the therapeutically required dose rates anyway, an accordingly higher activity must be used.

Suthanthiran (US 4,994,013) in his patent also describes cylindrical activity carriers consisting of a metallic substrate which is coated with carbon or active charcoal and contains the corresponding radionuclide. Instead of the carbon coating, US 5,163,896 mentions a material which consists of poly(amino acids).

Russell (US of 4,702,228) depicts palladium seeds in his patent. Therein, spherical aluminum compacts are described which contain enriched palladium-102. Using neutron activation, the Pd-102 is converted to Pd-103. The seed tube contains a spherical activity carrier in each of both ends. A rod-shaped X-ray marker is placed in between, which preferably consists of lead.

In a later patent (US 5,405,309), Carden discusses the disadvantages of Pd-102 neutron activation (dose rate difficult to adjust, undesirable isotopes as byproducts) and instead, suggests using Pd-103 free of carrier, produced in a cyclotron. The design is similar to that described by Russell, but instead of an aluminum carrier, graphite bodies are used therein which are coated electrolytically using an adjusted mixture of inactive palladium and palladium-103. In this design as well, the two activity carriers are situated at each end of the cylindrical seeds while the X-ray marker is positioned therebetween.

Such a design is disadvantageous in that a dose rate cannot be obtained in a substantial volume section of the seeds, namely, in the center thereof, because the X-ray marker is situated therein. Instead, the latter is responsible for a high degree of self-absorption, necessitating the use of an increased Pd-103 activity.

Because the use of palladium-103 in brachytherapy is advantageous compared to iodine-125 as a result of the short half-life and the more favorable radiation spectrum of the former, it was the object of the present invention to provide palladium-103 seeds for interstitial therapy which do not have the above-mentioned drawbacks and comply with the following requirements:
- more economical use of Pd-103 activity,
- minimizing self-absorption,
- homogeneous radiation field,
- improvement of spatial dose rate distribution,
- good visibility in X-ray diagnostics and identification of position,
- good abrasion resistance of the radionuclide,
- bulk production possible.

The object of the invention is accomplished by means of radioactive palladium-103 miniature radiation sources wherein the carrier matrix consists of an porous inorganic material, the pores of which contain radioactive Pd-103 as metal or in the form of a stable and water-insoluble metal compound. According to the invention, the production of the inorganic activity carriers can preferably be effected in an impregnation process (a) or in an injection molding or extrusion process (b).

The radioactive inorganic carriers thus produced are then encapsulated in or coated with a corrosion-resistant and body-compatible material. Physiologically tolerable materials which are suited for encapsulation or coating of the radioactive matrices are well known in the art. They may be composed of a resistant human tissue-compatible metal which also has low atomic weight to minimize X-ray shielding such as e.g. titanium or other corrosion-resistant metal alloy such as e.g. stainless steel.

Further, they may be composed of a resistant human tissue-compatible metal compound (using reactive nitrogen, oxygen, methane, or carbon monoxide gases during coating to form carbides, nitrides, or carbonitrides of transition metals or other metals) such as titanium carbide, titanium nitride, titanium carbonitride, titanium aluminum nitride, zirconium nitride, and hafnium nitride.

In a preferred embodiment of the present invention, the radioactive matrices are enclosed within a titanium or stainless steel capsule. Sealing of the capsule is effected using well-known methods, such as laser welding, TiG tungsten inert gas welding, or electron beam welding.

In a further embodiment, the materials for encapsulation or coating of the matrices may be composed of plastic materials which should be radiation-resistant, transparent to the radiation emitted and stable even in very thin layers as e.g. of 4-10 ìm. Such materials are well-known in the art and comprise polypropylene, polyethylene terephthalate, nylon, polyurethane, polyphenylene oxide blends, polyphenylsulfone, polysulfone, polyether sulfone, polyphenylene sulfide, phenyl ether ether ketone, polyether imide, and liquid-crystal polymer.

In a preferred embodiment of the present invention, Parylene® (poly-p-xylylene) may be used as encapsulation material or coating material. Similarly, polymer encapsulation is effected using well-known methods, such as introducing the inorganic activity carrier in a polymer hose and sealing same by means of gluing or welding.

The radioactive inorganic matrix may also be coated by the plastic material. In this case, the plastic is molded or shrunk onto the inorganic matrix, or the coating is formed by solvent evaporation or polymerization.

According to the present invention, as carrier matrix designated to carry the radioactive palladium-103, any porous inorganic material may be used which is mechanically stable and which does not absorb the low-energy gamma radiation of the palladium-103. Preferably, inorganic materials which have a porosity of about 15-40% are used, and particularly preferred is a porosity of about 20-30%. According to the invention the porous inorganic materials of the carrier matrix are selected from the group comprising ceramic materials, insoluble minerals, insoluble salts and inorganic polymers.

The ceramic materials, insoluble minerals, insoluble salts and inorganic polymers according to the invention are such materials which cations are selected from alkaline earth metals or their combinations (group 1), from Al, Si, Ga, Sn or their combinations (group 2) or from Ti, Zr, V, Cr, Mn, Fe, Co, Ni, Cu, Zn or their combinations and which anions form oxides, nitrides, carbides, phosphates, fluorides, sulfides or their combinations. It is also possible to use materials which comprise cations of groups 1 and 2, 1 and 3, 2 and 3 or of groups 1, 2 and 3.

Preferably used are for instance clays, porcelain, silicates or mixed metal oxides like zeolithes (Al/Si oxides), perovskites, spinels.

According to the invention especially preferred are ceramic materials, particularly such ceramic materials consisting of TiO₂, Al₂O₃, SiO₂, ZrO₂, Y₂O₃, mixtures thereof, glas or synthetic glas. It is particularly preferred to use Al₂O₃ or its mixtures with said other ceramic materials.

Due to the porosity of the inventive inorganic matrix and the included air, the seeds of the present invention already possess a good visibility in ultrasonics. If, additionally, the seed is provided with a X-ray marker, extremely good ultrasonic and fluoroscopic imaging is achieved. According to the invention, the materials of the X-ray markers are the common materials used for this purpose and well-known in the art, as for instance metals of high density, e.g. tantalum, tungsten or gold. In a preferred embodiment, gold is used. An additional improvement in ultrasonic imaging of the seeds can be achieved by coating the exterior surface of the covering with ultrasound echogenic coating, e.g. Echo Coat® or by roughening said surface.

The inorganic matrices of the invention may have any shape which is described in the prior art for radioactive carriers. They may be used in the form of e.g. a rod, hollow tube, film, sheet, microspheroidal particles or pellets. According to the invention, the tube-shaped inorganic matrices are preferred. The shape of the X-ray markers, of course, will depend on the shape of the inorganic matrix and on the location of the matrix within the capsule. The X-ray markers may be placed beside or around the inorganic matrix, or they may be placed centrally within the inorganic material. For example, the X-ray markers may be rotationally symmetrical elements, such as rods, tubes, beads, or hemispheres or rotationally symmetrical elements having a larger diameter at one or both ends and a lower diamter in the middle (between both ends) of the elements. The X-ray marker may consist of one or more pieces. The marker may occupy the whole or a partial length of the inside of the seed. According to the invention the marker indicates both position as well as orientation of the seeds.

As X-ray markers for tube-shaped inorganic carriers, markers in the form of a wire or tube and arranged centrally in the tube-shaped inorganic carrier are possible in a particularly preferred fashion. As X-ray markers for tube-shaped inorganic carriers of an exemplary length of 3.5 mm and an assumed outer diameter of 0.6 mm, wire cuts or tube cuts 0.3-4 mm in length and 0.1-0.7 mm in diameter, as well as beads 0.1-0.7 mm in diameter can be used, for example. Possible forms of markers for this purpose also include other rotationally symmetrical parts having overall lengths of 0.3-4 mm and diameters of 0.1-0.7 mm.

In an especially preferred embodiment of the invention, the X-ray marker is used in the form of a wire which is centrally placed within the inorganic matrix.

Preferably, the radioactive inorganic matrix, which is in a preferred embodiment of the invention tube-shaped, and the X-ray marker are inserted into a tube which is closed on one end, and then the other, originally open end of the tube is closed. In the case of a metal tube, the second end of the tube is sealed by laser-welding or any other suitable technique such as electron beam or tungsten inert gas welding.

In the case of a plastic tube, the second end of the tube is sealed by gluing, heat sealing, ultrasonic welding or solvent welding.

The radioactive inorganic matrix may also be coated by the plastic material. In this case, the plastic material is molded or shrinked onto the inorganic matrix or the coating is formed by solvent evaporation or polymerisation.

In one embodiment according to the invention, the production of the radioactive inorganic carriers can be effected using the impregnation process (a). In this process, the porous inorganic matrices are soaked with an aqueous solution of a palladium-103 compound either by adding said aqueous Pd-103 solution in portions to the inorganic carrier matrix using a pipette, for example, or by immersing the carrier matrix into the aqueous Pd-103 solution (batch method). The solution is distributed uniformly throughout the inorganic matrix and absorbed therein. Thereafter, the inorganic carrier is dried, to which end the inorganic carrier can be heated in order to speed up the drying process. This drying step is followed by a reduction step. Depending on the palladium compound employed, said reduction can be accomplished by tempering or by adding a gaseous or liquid reducing agent at significantly lower temperatures.

The procedure of soaking and reducing is repeated until the desired activity has been absorbed, which is determined by standard dose rate measurement.

According to the invention, any water-soluble Pd compound, e.g. Pd-103 salts such as, *inter alia*, Pd(NH₃)₄Cl₂, PdCl₂, PdBr₂, Pd(NO₃)₂, PdSO₄, Pd acetate, can be used as Palladium-103 compound. Likewise, Pd-103 complexes with other bases containing nitrogen or phosphorus, Pd-103 alcoholates, or Pd acetylacetonate can be used as palladium-103 compound in soaking the carrier matrix.

When using e.g. tetraamminepalladium-103 dichloride in loading the inorganic matrix, reduction is effected by tempering the loaded inorganic carrier at 400°C at minimum. The duration of tempering at 400°C depends on the concentration of compound applied and will be 30 minutes to 1 hour at minimum. Advantageously, the temperature increase to 400°C is effected stepwise, with 10 K/min being particularly preferred. During tempering, the tetraamminepalladium dichloride will decompose via the intermediate diamminepalladium dichloride to form metallic palladium. In this form it is insoluble and thus, cannot be eluted by aqueous solutions. In order to carry away gases possibly having formed, the tempering process is conducted advantageously in a stream of inert gas.

Alternatively, this tempering process can be replaced by a reduction process at significantly lower temperatures (about 60-150°C) in the presence of a reducing agent. For example, carbon monoxide or hydrogen are possible as reducing agents. When using hydrogen, it should be noted that the reduction has to be followed by subsequent tempering at about 800°C for 2 hours in order to decompose Pd-H adducts having formed.

When using, e.g., aqueous solutions of PdCl₂, PdBr₂, Pd(NO₃)₂, PdSO₄, Pd acetate, or complexes with other bases containing nitrogen or phosphorus, Pd alcoholates, or Pd acetylacetonate as palladium-103 compounds for impregnating, addition of a reducing agent in the reduction step is beneficial in order to avoid excessively high temperatures in the reduction. As described above, CO and H₂ are possible as gaseous reducing agents. Alternatively, the reduction may also be performed by adding reducing agents in aqueous solution, such as, e.g., hydrazine solution or formic acid. Metallic palladium will form as insoluble final product, which is finely dispersed in the inorganic matrix.

In another embodiment of impregnating using a Pd(NO₃)₂ solution, palladium oxide is formed in a subsequent tempering step (at about 500°C, 30 minutes), which likewise is sparingly soluble and thus, cannot be eluted under physiological conditions.

In another embodiment of the impregnating process, the carrier matrix may also be impregnated with one of the above-mentioned water-soluble palladium-103 compounds and subsequently loaded with a base such as NaOH, KOH, NH₄OH, so that palladium hydroxide will precipitate inside the pores, which then is converted to insoluble palladium oxide by subsequent tempering at about 400-500°C. In addition to impregnation using a base (e.g. 0.5 molar NaOH solution), precipitation reactions to form palladium fluoride or sulfide are also possible. In these cases, solutions containing fluoride or sulfide, such as NaF or NH₄HS, should be added to the carrier matrix soaked with one of the above-mentioned water-soluble palladium-103 compounds. This is followed by a tempering step wherein drying at about 200°C for about 30 minutes is effected. As a result, sparingly soluble palladium fluoride or sulfide is present in the pores of the carrier matrix.

As particularly preferred embodiments of the invention, variants are selected where Pd-103 is present in the carrier matrix in its final form as metallic Pd-103 which is stable and insoluble under physiological conditions.

A preferred embodiment of the impregnation process uses e.g. ceramic tubes as inorganic carriers. These tubes are commercially available. In a most preferred embodiment, the ceramic tubes are made of TiO₂, Al₂O₃, SiO₂, ZrO₂, Y₂O₃, or mixtures thereof, particularly Al₂O₃ or mixtures thereof with other ceramic materials.

The ceramic tubes to be used have a porosity of 15-40%. With a preferred length of e.g. about 3.5 mm, an outer diameter of about 0.6 mm and an inner diameter of 0.22-0.25 mm, there is sufficient pore volume to receive the required amount of activity.

As a rule, 0.2-4 mCi of Pd-103 has to be fixed on such a carrier. The precise values for each type of use are adapted in accordance with the dose rate requirements and the process and delivery times to be expected.

In a most preferred embodiment, tetraammine-Pd-103 dichloride [Pd(NH₃)₄Cl₂] is employed as palladium-103 solution in loading the inorganic carrier.

In order to produce e.g. Pd-103 miniature radiation sources having an activity of 1.9-2.1 mCi, a tetraammine-Pd-103 dichloride solution, for example, is prepared which has an activity concentration of preferably 5 mCi/µl at a pH value of 8-9. Then, small volumes of this solution, preferably 0.1 µ or 0.2 µl, are gradually pipetted on the inorganic carrier, the carrier is dried and subsequently, thermal reduction to form metallic palladium is effected, soaking and reducing being repeated until the entire solution has been absorbed and the carrier matrix has the desired activity. As a result, metallic Pd-103 is formed which is insoluble under physiological conditions and is situated in the pores of the carrier matrix.

The radioactive inorganic matrices can then be provided with a X-ray marker and encapsulated or coated with a material as described above.

Optionally, the X-ray marker may also be embedded within the inorganic matrix already at the beginning. The presence of the X-ray marker in the interieur of the matrix does not influence the activity loading process.

In a second embodiment of the invention, the inorganic activity carriers are produced in an injection molding or extrusion process (b).

In the injection molding process, the flowable inorganic mass, already containing the radionuclide, is forced with pressure into a mold where it solidifies, is removed from the mold, and subsequently tempered in the same way as in the impregnation process. Tempering is used to volatilize or burn out the hydrocarbon-containing plasticizing agent. Depending on the selected matrix, activity carriers of any shape can be obtained by using the injection molding process.

In addition to the injection molding method, an extrusion process may also be used wherein the inorganic mass, already containing the radionuclide, is forced through a die, the strand is collected, tempered in the same way as in the injection molding process, and finally cut to the desired length. Using the extrusion process, tube- or rod-shaped activity carriers can be produced.

According to the invention, the materials mentioned above are used as inorganic masses in the injection molding and extrusion processes, particularly metal oxide powders such as TiO₂, Al₂O₃, SiO₂, Y₂O₃, ZrO₂, or mixtures thereof, to which commercially available plasticizing aids are added to adjust the required viscosity and improve the sliding behavior. According to the invention, e.g. cellulose or a cellulose derivative in mixture with a polysaccharide and paraffin are used as plasticizing agent. It is preferred to use microcrystalline cellulose as cellulose. Preferably, 2 parts of cellulose and 3.3 parts of paraffin are used with one part by weight of polysaccharide. The amount of inorganic material depends on the size of the carrier to be produced and is between 200 and 250 mg for the tube-shaped activity carrier preferred according to the invention, the final dimensions of which being a length of about 3.5 mm, an outer diameter of about 0.6 mm, and an inner diameter of 0.22-0.25 mm. The specified components are mixed and, with addition of water, a homogeneous mass is produced. Thereafter, the radionuclide is added in such a way that an aqueous solution of one of the above-mentioned palladium-103 compounds, most preferably tetraamminepalladium-103 dichloride solution ([¹⁰³Pd(NH₃)₄]Cl₂), is added with a pipette, and stirred and homogenized during addition. The added solvent volume must be taken into account with respect to the amount of solvent employed in total.

Following thorough mixing, the mass is injected or extruded into the prepared mold, to which end machinery with miniaturized sample volumes are to be used as injection molding machines or extruders. The molds for the injection molding process are of such a design that, as a result, activity carriers in the form of tubes, rods, pellets, films, beads, or other molded bodies are obtained.

Injection molding is preferably performed at about 70°C as low-pressure hot injection molding. The cooled inorganic carriers are then removed from the mold and placed in an oven for tempering. In extruding, the extruded product is placed on a ceramic backing. In a temperature program wherein the temperature is increased stepwise to at least 400°C, preferably 400-500°C, the parts obtained in injection molding or extruding are subjected to annealing in order to drive out the plasticizing agents on the one hand, and, on the other hand, initiate the decomposition of the palladium-103 compound contained in the mass, to form metallic palladium.

To this end, when using [¹⁰³Pd(NH₃)₄]Cl₂ as Pd compound, temperatures of at least 400°C must be reached and maintained for at least 30 minutes to 1 hour, depending on the concentration of the compound. In a preferred embodiment, the temperature is increased in steps of 5 K/min. The process is conducted in a stream of inert gas in order to carry away the reaction gases. The tetraamminepalladium dichloride preferably used as palladium-103 compound undergoes decomposition via the intermediate diamminepalladium dichloride to form metallic palladium. In this form, it is insoluble and thus, cannot be eluted by aqueous solutions.

When using other Pd compounds such as ¹⁰³PdCl₂, reannealing at about 800°C for 30 minutes is required subsequent to the above-described tempering in order to obtain metallic, finely divided palladium from these compounds as well.

Alternatively, the tempering process can be replaced by a reduction process at significantly lower temperatures (about 60-120°C) in the presence of a reducing agent. For example, carbon monoxide or hydrogen are possible as reducing agents.

When producing the tube-shaped inorganic activity carriers which are preferred according to the invention, the final dimensions are understood to be the following: The length is preferably about 3.5 mm, the outer diameter is about 0.6 mm, and the inner diameter is 0.22-0.25 mm.

As a rule, 0.2-5 mCi of Pd-103 has to be fixed per carrier. The precise values for each type of use are adapted in accordance with the dose rate requirements and the process and delivery times to be expected.

Due to the tempering of the injection-molded or extruded tube, shrinking will occur during this step. Accordingly, the injection molds and extrusion dies are designed so as to be larger. Varying activity contents of Pd-103 have little effect on said shrinking, so that this factor does not have to be observed.

In a most preferred embodiment, tetraamminepalladium-103 dichloride is used as palladium-103 compound for injection molding or extruding. To produce the tube-shaped inorganic carriers which are preferred according to the invention, a solution is prepared from said compound, which has an activity concentration of 0.5-10 mCi/µl at a pH value of 8-9.

The inorganic carriers produced according to one of the methods of the invention can be provided with an X-ray marker and are then encapsulated in a body-compatible material such as titanium or stainless steel. The X-ray markers can be deposited in the inorganic material before or after injection molding or extruding.

Thus, according to the invention, palladium-103 miniature sources can be produced with those sizes and activities as required for interstitial brachytherapy where, in particular, the outer diameter, i.e., the diameter of the external tube enclosing the radioactive carrier matrix should not exceed 0.8 mm.

In a particularly preferred embodiment of the invention the length of the outer encapsulating tube may vary from 1 to 5 mm, the optimal value is about 4.5 mm. The wall thickness may vary from 0.010 mm to 0.150 mm if titanium is used and from 0.002 mm to 0.150 mm if a plastic material is used for encapsulation. The optimal thickness for titanium is about 0.050 mm, for parylene® coating from 10-20 µm.

The outer diameter of the carrier matrix corresponds to the inner diameter of the encapsulating tube. For a stable manufacturing process (insertion of the matrix into outer tube) the optimal outer diameter of the carrier matrix is 0.100 mm less than the inner diameter of the encapsulating tube.

The length of the x-ray marker may vary from 0.1 mm to the total length of the inner of the tube. The diameter ranges from 0.1 mm to 0.5 mm, whereas the optimal diameter is between 0.15 and 0.25 mm. The optimal length is about 3.5 mm.

The inner diameter of the carrier matrix corresponds to the diameter and shape of the x-ray marker. In case the marker is mechanically inserted into the tube-shaped activity carrier there should be a difference of 0.05 mm in the diameters.

The inorganic carrier matrices of the invention were found to have good abrasion resistance because the radioactive palladium is not present as a layer on a smooth surface but rather, in the pores of the inorganic carriers. The inorganic carriers produced according to the invention are mechanically stable and thus, allow good handling during loading and mounting. Compared to other variants in design as described in prior art, the inorganic carriers according to the invention exhibit a significantly more homogeneous dose rate distribution. Particularly in that case where the X-ray marker is present as a wire and thus, fills not more than a fraction of the inner diameter, lower self-absorption is observed and therefore, a lesser use of activity is required. Consequently, the sources according to the invention can be produced in a more cost-effective way. In a preferred embodiment of the invention the X-ray marker is as thin as possible, preferably in the form of a wire, reaching exactly from one end to the other at the inner of the rod or tube or pellet.

In the process of the invention, the X-ray marker preferably is incorporated at the end of the process. In this way, variations in diameter or length are possible. However, the marker may also be incorporated as early as at the beginning of the process. Thus, in the injection molding process, for example, the X-ray marker can be put in first and enclosed tightly by the injected mass. In this event, however, care must be taken to select those reduction processes where the temperatures do not exceed 150°C because otherwise, fractures and cracks in the inorganic activity carrier might occur as a result of the dissimilar heat expansion coefficients.

With reference to the embodiments which are not intended to be limiting, the invention will be illustrated in more detail below.

### Example 1

### Production of Pd-103 miniature radiation sources having an activity of 0.45-0.55 mCi using the impregnation process (pipette method)

Al₂O₃ ceramic tubes having an outer diameter of 0.60 + 0.02 mm, a length of 3.5 + 0.05 mm, and an inner diameter of 0.21 + 0.01 mm are used. 200 of these are placed on a rack in prefabricated, cut-out positions in a well-defined fashion. It is made sure that each position can be reached by an automatic pipette.

Starting with a [¹⁰³Pd(NH₃)₄]Cl₂ stock solution, a solution is adjusted which has an activity concentration of 2.5 mCi/µl. Using a computer-controlled movable pipette, each ceramic is initially soaked with 0.1 µl of solution. Following air-drying, each ceramic is soaked again with 0.1 µl of solution. Thereafter, the rack is moved into an oven wherein the following temperature program is started: starting with 50°C preheating, to 400°C with 10 K/min, then hold for 30 minutes and allow to cool. The oven is operated in a stream of inert gas.

Subsequently, the ceramic activity carrier is taken out of the rack, filled into a titanium tube sealed on one end, the X-ray marker is threaded, and laser-welding is performed.

### Example 2

### Production of 100 pieces of Pd-103 miniature radiation sources having an activity of 2.9-3.1 mCi using the batch impregnation process

Similarly, Al₂O₃ ceramic tubes having an outer diameter of 0.60 + 0.02 mm, a length of 3.5 + 0.05 mm, and an inner diameter of 0.21 + 0.01 mm are used. 100 of these are placed in a cylindrical vessel having a screen bottom.

Starting with a [¹⁰³Pd(NH₃)₄]Cl₂ stock solution, a solution is adjusted which has an activity concentration of 5 mCi/µl. 0.5 ml of this solution is used to completely impregnate the previously placed ceramics. The supernatant flows into a collecting vessel. Any solution adhering to the ceramics is sucked off by briefly applying a vacuum. Following air-drying, the ceramics are transferred into an oven equipped with a controlled gas supply and a gas outlet. Under a continuous stream of carbon monoxide gas, the ceramics are tempered at 150°C for 10 minutes and subsequently removed.

Now, impregnating and subsequent tempering are performed once again, and this process is repeated until the activity carriers have reached the target activity. The latter is determined by measuring the dose rate. Thereafter, the ceramic activity carriers are removed, filled one by one into a titanium tube sealed on one end, the X-ray marker is threaded, and laser-welding is performed.

### Example 3:

### Production of 100 pieces of Pd-103 miniature radiation sources according to the invention, each having an activity of 5 mCi, using the injection molding process

The flowable mass required for the injection molding process is produced as follows:
220 mg of aluminum oxide having an average grain diameter of 1-5 ìm is placed in a miniature stirred vessel heated to about 70°C, mixed with 12 mg of cellulose powder (microcrystalline cellulose), 6 mg of starch is added, mixed by stirring, and eventually, 20 mg of paraffin is added. Finally, water is added until a coarsely flowable mass of a paraffin-water emulsion is formed which can be transferred without residue into the likewise heated sample chamber of the miniature injection molding machine. Now, a total of 50 µl of a tetraamminepalladium-103 dichloride solution having an activity concentration of 10 mCi/µl and a pH value between 8 and 9 is pipetted into the sample chamber, with stirring being effected after each addition. Following addition of all the aliquots, the remaining volume of water is added.

Following thorough homogenization, the process of low-pressure hot injection molding is performed at last, wherein the mass is forced into a cold mold where it solidifies, is removed from the mold, and transferred into an oven. Tempering is used to volatilize or burn out the hydrocarbon-containing plasticizing agent, but at the same time, bring about reductive decomposition of the tetraamminepalladium-103 dichloride to form metallic, finely divided palladium-103. In order to carry away reaction gases having formed, the oven is operated in a stream of inert gas. The following temperature program is performed: from room temperature to 500°C with 5 K/min, then hold at final temperature for 60 minutes and allow to cool.

Eventually, the radioactive ceramic carriers are provided centrally with an X-ray marker and encapsulated in a body-compatible material such as titanium or stainless steel. To this end, the radioactive ceramic carrier including the X-ray marker is preferably introduced in a tube sealed on one end, and the remaining open end is welded using a laser.

The features of the invention will be better understood from the following description of a most preferred embodiment with reference to the accompanying drawing(s) in which:
Fig. 1 is a sectional view of a tube-shaped seed in accordance with the invention;
Fig. 2 is a longitudinal sectional view of the seed according to Fig. 1.

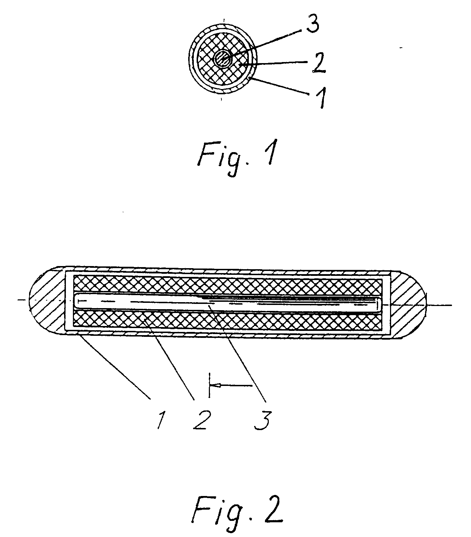

In Fig. 1, there is shown a sectional view of a seed having a porous inorganic tube 2, the pores of which contain the radioactive palladium-103. The inorganic tube 2 is surrounded by a capsule 1. The capsule 1 has an outer diameter of less than 1 mm. Inside the hollow space of the tube 2, an X-ray marker 3 is centrally arranged.

Fig. 2 shows the seed in a longitudinal sectional view. The X-ray marker 3 is a wire which extends from one end of the inorganic tube 2 to the other.

The capsule 1 is made of titanium. The two ends of the capsule 1 are laser-welded after inserting the inorganic tube 2 including the X-ray marker 3 into the capsule 1.

## Claims

1. A medical radioactive palladium-103 miniature radiation source which is enclosed in a corrosion-resistent and body-compatible material, which is transparent for the emitted radiation and resistant to said radiation wherein the carrier matrix consists of a porous and mechanically stable inorganic material, the pores of which contain palladium-103 as metal or in the form of a stable and water-insoluble Pd-103 compound.

2. The Pd-103 miniature radiation source according to claim 1, characterized in that the inorganic material of the carrier matrix has a porosity of 15-40%, preferably 20-30%.

3. The Pd-103 miniature radiation source according to claim 1 or 2, characterized in that the porous inorganic material of the carrier matrix is selected from the group comprising ceramic materials, insoluble minerals, insoluble salts and inorganic polymers, preferably ceramic materials.

4. The Pd-103 miniature radiation source according to any of claims 1 to 3, characterized in that the ceramic materials consist of TiO₂, Al₂O₃, SiO₂, ZrO₂, Y₂O₃, mixtures thereof, glass or synthetic glass, preferably Al₂O₃ or its mixtures with said ceramic materials.

5. The Pd-103 miniature radiation source according to any of claims 1 to 4, characterized in that the pores of the carrier matrix contain palladium oxide, palladium sulfide or palladium fluoride as Pd-103 compound.

6. The Pd-103 miniature radiation source according to any of claims 1 to 5, characterized in that the miniature radiation source includes one or more X-ray markers made of a high-density metal, preferably tantalum, tungsten or gold.

7. The Pd-103 miniature radiation source according to any of claims 1 to 6, characterized in that the carrier matrix has the form of a tube, rod, film, or pellet, or a spherical form, preferably a tube form.

8. The Pd-103 miniature radiation source according to any of claims 1 to 7, characterized in that the X-ray marker is arranged in the carrier matrix or adjacent to the carrier matrix, or surrounds the carrier matrix.

9. The Pd-103 miniature radiation source according to any of claims 1 to 8, characterized in that the X-ray marker is arranged centrally in the carrier matrix.

10. The Pd-103 miniature radiation source according to any of claims 1 to 9, characterized in that the X-ray marker is a wire or a tube.

11. The Pd-103 miniature radiation source according to any of claims 1 to 10, characterized in that a wire or a tube as X-ray marker is centrally arranged in a tube-shaped carrier matrix.

12. The Pd-103 miniature radiation source according to any of claims 1 to 11, characterized in that the material in which the carrier is enclosed is a metal alloy, or a plastic material.

13. A process for the production of a medical radioactive palladium-103 miniature radiation source, characterized in that a porous inorganic carrier matrix is soaked with an aqueous solution of a palladium-103 compound and, following drying of the carrier matrix, the employed water-soluble Pd-103 compound in the carrier matrix is reduced to form metallic Pd-103 or converted to a water-insoluble Pd-103 compound, and the resulting radioactive inorganic carrier matrix is enclosed in a corrosion-resistent and body-compatible material.

14. The process according to claim 13, characterized in that the aqueous solution used to soak the carrier matrix contains a Pd-103 salt, a Pd-103 complex with bases containing nitrogen or phosphorus, a Pd-103 alcoholate, or Pd-103 acetylacetonate as palladium-103 compound.

15. The process according to claim 14, characterized in that PdCl₂, PdBr₂, Pd(NO₃)₂, PdSO₄, Pd acetate, or [Pd(NH₃)₄]Cl₂ is used as Pd-103 salt.

16. The process according to any of claims 13 to 15, characterized in that the soaking of the carrier matrix with the aqueous solution of a palladium-103 compound is effected using a pipette, or by immersing the carrier matrix into the aqueous solution.

17. The process according to any of claims 13 to 16, characterized in that a [Pd(NH₃)₄]Cl₂ solution is used as Pd-103 compound and, following soaking and drying of the carrier matrix, reduction to form metallic Pd-103 is effected by tempering at 400°C at minimum.

18. The process according to any of claims 13 to 17, characterized in that the reduction to form metallic Pd-103 is performed in the presence of a gaseous reducing agent or an aqueous solution of a reducing agent at temperatures of about 60-150°C.

19. The process according to any of claims 13 to 16, characterized in that a Pd(NO₃)₂ solution is used as palladium-103 compound and, following soaking and drying of the carrier matrix, conversion to form palladium oxide is effected by tempering at about 500°C.

20. The process according to any of claims 13 to 16, characterized in that following soaking of the carrier matrix with an aqueous solution of a Pd-103 compound according to claim 13, a strongly basic solution is applied to the carrier matrix so as to form Pd(OH)₂ which is converted to palladium oxide in a subsequent tempering at about 400-500°C.

21. The process according to any of claims 13 to 20, characterized in that a tube-shaped matrix is used as carrier matrix.

22. A process for the production of a medical radioactive palladium-103 miniature radiation source, characterized in that a homogeneous mass is prepared using an inorganic material least one metal oxide powder, to which a plasticizing aid has been added, said mass is added with an aqueous solution of a Pd-103 compound and mixed homogeneously, the obtained mass is injection-molded or extruded to the desired shape of the radioactive carrier matrix and subsequently, the employed Pd-103 compound in the carrier matrix is reduced to form metallic Pd-103 or converted to a water-insoluble Pd-103 compound, and the resulting radioactive inorganic carrier matrix is enclosed in a corrosion-resistent and body-compatible material.

23. The process according to claim 22, characterized in that injection molding is performed as low-pressure hot injection molding at about 70°C.

24. The process according to claim 22 or 23, characterized in that the reduction to form metallic Pd-103 or the conversion to form a water-insoluble Pd-103 compound is effected by tempering or in the presence of a reducing agent according to claims 17-19.

25. The process according to any of claims 22 to 24, characterized in that metal oxide powders are used as inorganic material, preferably TiO₂, Al₂O₃, SiO₂, ZrO₂, Y₂O₃, or mixtures thereof, preferably Al₂O₃ or mixtures thereof are used as metal oxide powder.

26. The process according to any of claims 22 to 25, characterized in that the injection-molded or extruded radioactive carrier matrix obtained has the shape of a tube.

27. The process according to any of claims 13 to 26, characterized in that a X-ray marker, preferably a wire is incorporated centrally in the radioactive carrier matrix before enclosing it in a corrosion-resistent and body-compatible material.
